Europäisches Patentamt

European Patent Office

Office européen des brevets

(11) Publication number: **0 288 576 B1**

## EUROPEAN PATENT SPECIFICATION

(45) Date of publication of patent specification: **03.02.93**

(51) Int. Cl.⁵: **A61B 17/36, A61L 29/00, A61M 25/00**

(21) Application number: **87906945.8**

(22) Date of filing: **27.10.87**

(86) International application number:
**PCT/JP87/00825**

(87) International publication number:
**WO 88/03005 (05.05.88 88/10)**

(54) **BALLOON FOR ENDOSCOPE.**

(30) Priority: **29.10.86 JP 259595/86**
**18.06.87 JP 153105/87**
**18.06.87 JP 153106/87**
**19.06.87 JP 94141/87 U**
**22.06.87 JP 156016/87**
**22.06.87 JP 156017/87**
**19.06.87 JP 94142/87 U**
**19.06.87 JP 94143/87 U**
**19.06.87 JP 94144/87 U**
**19.06.87 JP 94145/87 U**
**22.06.87 JP 96056/87 U**
**22.06.87 JP 96057/87 U**
**22.06.87 JP 96058/87 U**

(43) Date of publication of application:
**02.11.88 Bulletin 88/44**

(45) Publication of the grant of the patent:
**03.02.93 Bulletin 93/05**

(84) Designated Contracting States:
**FR IT**

(73) Proprietor: **OKAMOTO INDUSTRIES, INC.**
**27-12, Hongo 3-chome**
**Bunkyo-ku Tokyo 113(JP)**

Proprietor: **M & M CO., LTD.**
**19-2, Iriya 1-chome**
**Taitho-ku Tokyo 110(JP)**

(72) Inventor: **SHIMAMURA, Yoshiyuki**
**128-1, Takatsukashinden**
**Matsudo-shi Chiba 271(JP)**
Inventor: **TSUSHIMA, Kyogo**
**144-1, Itabashi-cyo**
**Ryugasaki-shi Ibaragi 301(JP)**
Inventor: **SETO, Toshihito**
**1-1-508, Hikarigaoka**
**Nerima-ku Tokyo 176(JP)**

(74) Representative: **Lehn, Werner, Dipl.-Ing. et al**
**Hoffmann, Eitle & Partner Patentanwälte Arabellastrasse 4**
**W-8000 München 81(DE)**

(56) References cited:
**EP-A- 0 101 012**      **EP-A- 0 101 012**
**EP-A- 0 144 132**      **WO-A-83/03188**
**DE-A- 3 406 294**      **FR-A- 2 302 755**
**JP-A- 6 173 644**      **JP-A-61 193 655**
**US-A- 2 188 736**

## Description

This invention relates to medical instruments for observing, diagnosing and curing diseased parts within blood vessels or tubes such as the gullet, the stomach, the duodenum, the colon, the nasopharynx, the cystic duct, the abdominal cavity, the bladder, the uterine neck and the like, particularly to instruments of medical treatment by laser beam, and more particularly it relates to an endoscope or balloon for using optical fiber according to the preamble of claim 1.

In the past, such a balloon for use with an endoscope or an optical fiber has not been widely known. Therefore, in the case of treating some diseased part within blood vessels or internal tube organs by means of laser surgery, such an endoscope or optical fiber is firstly inserted into the blood vessels or the internal tube organs to illuminate the interior of a diseased part by a light-guide which is attached to the end portion of the endoscope to allow for the observation or diagnosing of the location of the diseased part or internal hemorrhage through an object lens of the endoscope. After that, the laser surgery was conducted with the optical fiber which was inserted therein.

However, the head attachments such as the light-guide, the object lens and/or the optical fiber are exposed at the top end so that these were easily adhered to and stained by blood, body fluids, foods, sarcomata, secreting fluid and the like. Consequently, the diseased parts were often invisible or the irradiation of the laser was insufficient. Furthermore, as a result of erosion on the surfaces of the attachments caused by the adherence of the above, a danger of impairing the functions of the attachments exists. Additionally, when the endoscope is inserted into the blood vessels as in the above case, apart from the original inserting location of the endoscope, one or two other locations in the blood vessels must first be cut open and disposable type tube catheters suitable for use in the blood vessels are inserted into the blood vessels in these additional openings to inject a quantity of a physiological salt solution through the catheter into the blood vessels in order to inflate the blood vessels with the solution for stopping the flow of blood temporarily. The endoscope is then inserted through the proper opening.

As to the material of the catheter, it is not transparent, so that transillumination of the laser beam through the tube wall does not occur.

Furthermore, in the case of general transparent rubber products, these materials have a tendency to decrease in strength because of the use of a small amount of powder type vulcanizing agent or vulcanizing accelerator. Therefore, in order to maintain the strength of the material, it was common to increase the thickness of material, which in turn meant that it could not be inflated.

From WO-A-83/03188, an endoscopic device is known which includes a balloon comprising an elastic transparent thin film body incorporating a hollow globular inflatable portion formed on one end of a cylindrical fixing portion.

It is an object of the present invention to provide a balloon for an endoscope for using an optical fibre which prevents scattering of broken pieces in the event of the balloon bursting.

This object is solved in accordance with the present invention by a balloon having the features of claim 1.

This invention provides a body having a transparent film which forms an inflating portion on the head of a fixing portion for fixing the body on the end portion of the endoscope or the flexible protecting tube of the optical fiber such that the inflating portion is fixed coveringly and projectedly onto said end portion. The inflating portion is inflated by means of injecting a quantity of physiological salt solution therein. Furthermore, the inflating portion is transilluminative by the laser beam as a result of its transparency. By fixing the body having the thin film on the end portion of the endoscope, the light-guide, the object lens, the optical fiber and attachments are protected from the adherences thereto of blood, secreting fluid and the like, so that each respective function can be effectively and sufficiently carried out.

In accordance with the invention, a protective film body is provided on the inflating portion as a second skin and prevents the breakdown of the balloon on bursting of the inflating portion caused by pricking with the injection needle into the inflating portion when injecting medical liquids or using the injection needle for medication, when using the optical fiber while irradiating the laser beam. Furthermore, even if the inflating portion is broken, this protective film can still prevent the scattering of the broken pieces within the blood vessel or organs thereon, whereby the inflating portion then finally shrinks therein.

Furthermore, as the inflating portion is inflated by means of the physiological salt solution being injected from the ports supplying air and water, a predetermined space is provided which widens the visual field so that the observation and diagnosis or treatment is made easier and can be performed with more certainty. Therefore, for example, diagnosis of focuses of thrombus and the like by means of irradiation of the laser beam at the focuses can be made, or pressure can be applied upon the focuses of the gullet varix or upon the focuses around the duodenum to check bleeding from the focuses. Otherwise, it is also possible to widen the strangulated focuses and cure them.

Furthermore, the various laser beams such as YAG (Yittrium, Aluminium, Gallium) laser, Argon laser, Argon dye laser and the like, which are transilluminated through the optical fiber, can be irradiated into other focuses in each internal organ, thereby checking bleeding or cauterizing the focuses for curing them. It is also possible to give an injection of medical fluids into the diseased portion. Thus, the invention is useful for providing new treatments.

In addition, as a result of being able to stop the blood flow within the blood vessel, it is neither necessary to operate another opening upon the blood vessel as in the past nor to insert another catheter for stopping the blood flow.

According to a development of the invention, the body having the thin film is formed with a globular shaped inflating portion in the same body by means of a dipping process. In this process, the inflating portion is formed by means of a waist portion with one side of a cylindrically fixed portion, whereby the film thickness of the waist portion is provided with a shape thicker than the film thickness of the fixed portion and inflating portion. This forming process of the body is extremely advantageous. Furthermore, the waist portion displays a strangle-hold-effect in contrast to the effect when the globular shaped inflating portion is inflated. Therefore, the inflating portion positioned at the end portion of the waist portion may be inflated easily.

The balloon body having the transparent thin film which forms the inflating portion is made in a manner whereby the forming mold of the above is dipped into a composite solution which contains 100 WF (weight fraction) of the rubber content of rubber latex with additives of 0.7--1.5 WF of powdered vulcanizing agent, 0.2--0.5 WF of powdered vulcanizing accelerator, 0.5--2.0 WF of fluid granulating accelerator and 0.5--2.0 WF of fluid type antioxidant. The product formed in the mold is then dried by a vulcanizing pan. Accordingly, such a thin film body having transparency and intensity is obtainable which furthermore prevents losing sight of the focuses owing to its transparency, and, in accordance with the excellent transmissivity for the laser beam, excellent curing effects can be achieved by the irradiation of the laser beam.

The accompanying drawings show several embodiments of a practical example of the balloon for the endoscope or the optical fiber of the invention wherein,

Fig. 1 is a longitudinal elevational view of a conventional balloon,

Fig. 2 and Fig. 3 are cross sectional views respectively along line (2)-(2) and line (3)-(3) of Fig. 1,

Fig. 4 is a schematic illustration in which the thin film body is fixed to the end portion of the endoscope,

Fig. 5 is a schematic illustration in which the conventional balloon with the endoscope is inserted into the blood vessel wherein the inflating portion of the body is inflated,

Fig.6 to 8 show various possible shapes of conventional balloons,

Fig 9 to 11 show a balloon according to the present invention,

Fig. 12 and Fig. 13 inclusively show further practical examples of conventional balloons,

Fig. 14 is an elevational view of the end portion of a conventional endoscope,

Fig. 15 and Fig. 16 are schematic illustrations of further practical examples in which the bodies of balloons are fixed with the end portions of the endoscopes,

Fig. 17 is a longitudinal elevational view showing the body fixed to the flexible protective tube for an optical fiber;

Fig. 18 is a sectional view along to line (18)-(18) of Fig. 17,

Fig. 19 is a sectional view with all parts disassembled and

Fig. 20 is an enlarged view of the body inserted into the blood vessel wherein the inflating portion is inflated.

Equivalent features of the conventional and inventive balloons are denoted with the same reference signs throughout.

Referring now in detail to the drawings as to the several embodiments of the practical examples showing conventional and inventive balloons, the body (A) is formed by the transparent thin film, having a thickness of 0.10 to 0.40 mm inclusive, which consists of rubber latex, silicone rubber and the like, wherein the inflatable inflating portion (2) having an elasticity is integrally formed with the end of fixing portion (1) which is furthermore fixed insertingly with the end portion (b) of endoscope (B). The body (A) is inserted into the end portion (b) as the inflating portion (2) is projected from the end portion (b).

The fixing portion (1) is of a cylindrical shape, having a diameter of approximately 1.0 to 17.0 mm inclusive. One side end thereof is shaped to be opened and furthermore has a shape in sectional view of a circle, an ellipse, a triangle, a square or other polygons and the like as optional shapes, and in compliance with requirements, shapes of a projection, a hollow portion, a concave-convex shaped groove and the like are also applicable. The inflating portion (2) is integrally provided with the fixing portion such that when the end portion (b) of the endoscope (B) is inserted into within the fixing portion (1) through the aforesaid opened portion, the inflating portion (2) projects from the end edge of the endoscope (B). The fixing portion (1) which covers the end portion (b) may be attached by optional means such as stitches or by using a bond for fixing the portion (1). In this case, it is advantageous to provide anti-slip concave-convex portions on the outer surface of the end portion (b).

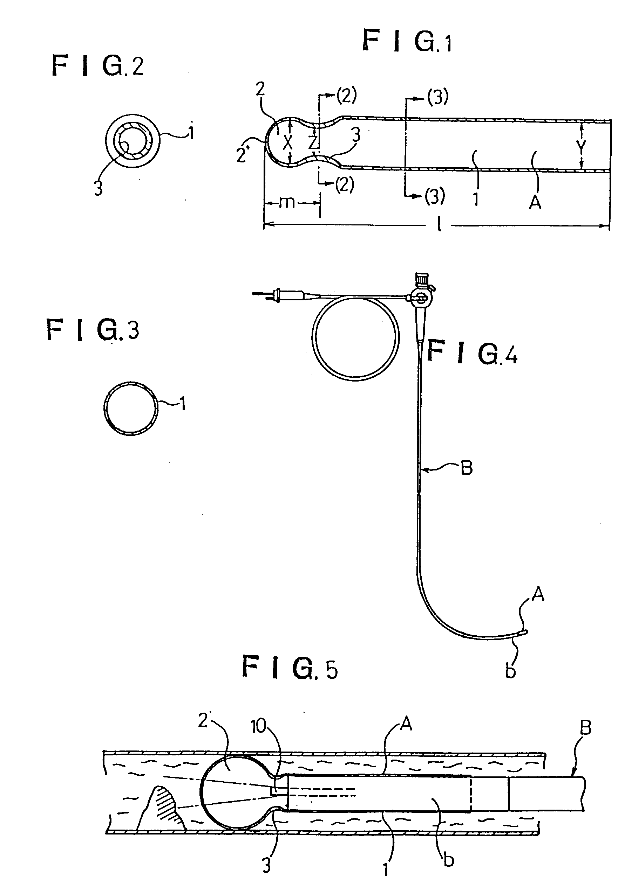

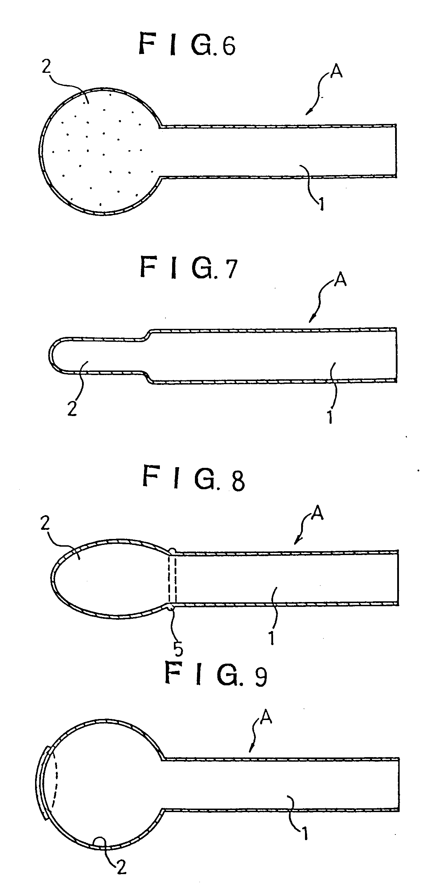

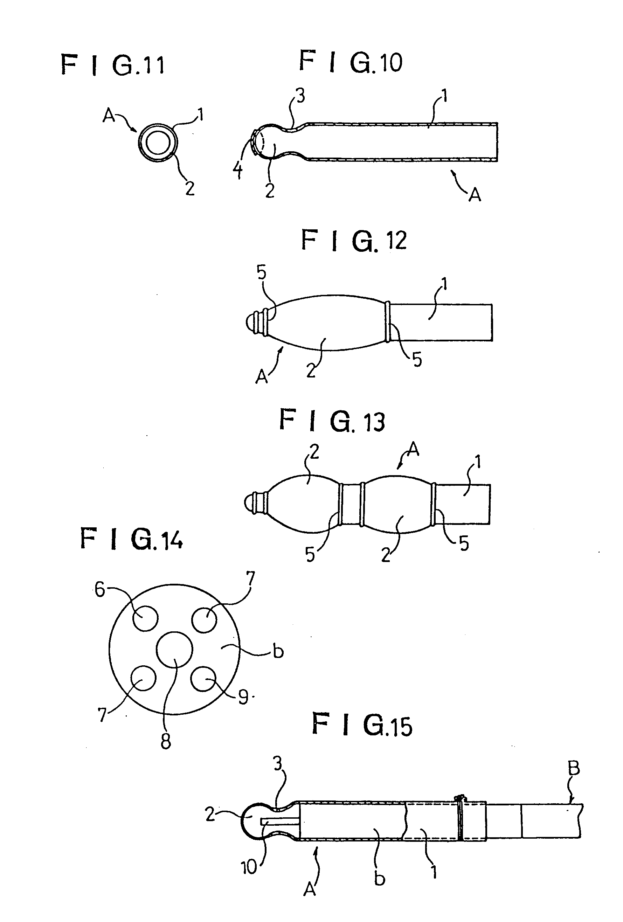

The inflating portion (2) formed on one end of the fixing portion (1) is composed of an elastic globular body (Fig. 1 and Fig. 6), or of a projecting inflated body (Fig. 8) or a cylindrical body (Fig. 7). In the case where the body (A) is fixed covering the end portion (b) of the endoscope (B), the body (A) is inflated to a predetermined size by means of physiological salt solution injected therein from the channel of the endoscope projecting from the end, or from the air and water supplying orificies attached thereto. In its inflated condition, the body (A) has a shape similar to that prior to being inflated. It is optional as to whether the body (A) is formed with a diameter larger than the diameter of fixing portion (1) or not (Fig. 6 and Fig. 8). It is mostly formed with a diameter the same as that of the fixing portion but also in combination of shape and diameter.

The diameters of body (A) may be of 0.9 to 35.0 mm inclusively, that is, it is preferably in compliance with the diagnosis and the way of curing. In any case, as the thickness of the body film is decreased as a result of the inflow of physiological salt solution for inflating the body, the thin film of the body not only becomes sufficiently transmissive, but lasers such as YAG laser, the argon laser, the argon dye laser and the like are also transilluminated effectively.

The inflating portion (2) has the additional function of arresting bleeding from a bleeding portion or the focuses of diseased parts by means of the compression produced by the inflation. Also, to make the isthmuses of blood vessels such as the loop blood vessel surrounding the heart to widen, the inflating portion is inflated to make full contact with the interior wall of the blood vessel and therefore closes this and blocks the blood flow.

The thin film of rubber latex, which has excellent elasticity and is composed of a transparent thin film with a thickness of 0.10 to 0.40 mm inclusive, is formed into the fixing portion (1) and inflating portion (2) when it is dried after the rubber latex is adhered to the forming mold having the same shape as the body (A). In this process, the mold is dipped into the composed solution in such a manner that the inflating portion faces downwards The composed solution consists of 100 WF of the rubber latex with additives of 0.7--1.5 WF of powdered vulcanizing agent, 0.2--0.5 WF of powdered vulcanizing accelerator, 0.5--2.0 WF of fluid granulating accelerator and 0.5--2.0 WF of fluid antioxidant. There are some cases where the head portion (2') of inflating portion (2) has a thicker film shape.

The basic concept of this invention lies in that, the transparent thin film body (A) being formed incorporatedly with the inflatable portion (2) at the end of fixing portion (1) as described above is provided such that a protective film body (4) is applied to the end of the inflatable portion (2) remote from the fixing portion (1) (Fig. 9 and 10). However, in accordance with an embodiment of the invention, there can be provided a waist portion (3) between the fixing portion (1) and the inflating portion (2) (Fig. 1 and Fig. 10), or both the waist portion (3) and the protective film body (4) can be provided together (Fig. 10).

The waist portion (3) is a smaller diameter portion which is formed between the cylindrical fixing portion (1) and the globular shaped inflating portion (2), and which has 75--85% (average 80%) of the diameter of the fixing portion, wherein the film thickness of the waist portion can be formed slightly thicker than the fixing portion and inflating portion.

With reference to Fig. 1, the following tables show the mutual relationships of film thickness, the interior diameter of each of (X), (Y) and (Z), the whole length (1) and the length of globular body (m) in the cylindrical fixing portion (1), the globular inflating portion (2), the head portion (2') and the waist portion (3), wherein several examples are shown below, although these figure are not restrictive;

(Film thickness: m.m.)

| Examples | (1) | (3) | (2) | (2) |
|---|---|---|---|---|
| No. 1 example | 0.18 | 0.25 | 0.19 | 0.22 |
| No. 2 " | 0.16 | 0.22 | 0.16 | 0.19 |
| No. 3 " | 0.16 | 0.22 | 0.19 | 0.19 |
| No. 4 " | 0.15 | 0.21 | 0.20 | 0.20 |
| No. 5 " | 0.16 | 0.22 | 0.18 | 0.20 |
| No. 6 " | 0.18 | 0.22 | 0.20 | 0.21 |

(Inside diameter & length: m.m.)

| Examples | (Y) | (Z) | (X) | m | l |
|---|---|---|---|---|---|
| No. 1 example | 1.50 | 1.25 | 1.40 | 1.39 | 10.5 ± 10% |
| No. 2 " | 1.94 | 1.54 | 1.81 | 1.96 | 13.0 ± 10% |
| No. 3 " | 3.00 | 2.46 | 2.85 | 3.16 | 17.0 ± 10% |
| No. 4 " | 5.97 | 4.45 | 5.43 | 5.22 | 26.0 ± 10% |
| No. 5 " | 12.01 | 10.31 | 11.32 | 12.01 | 34.0 ± 10% |
| No. 6 " | 16.92 | 14.01 | 15.26 | 14.81 | 40.0 ± 10% |

The inventive anti-breakdown protective film (4) which is formed in the shape of a thin film on the inflating portion (2) is composed of synthetic rubbers such as rubber latex with the same quality of the inflating portion (2) or the silicon rubber, CR, SBR and the like which are applied or adhered to the head surface (2') of the inflating portion (2). The protective film (4) has the specific characteristics such as excellent adhesiveness to the inflating portion (2) and an elasticity similar to the inflating portion (2) to prevent any exfoliation of the film (4) from its base. The protective film (4) is applied or adhered preferably to the head surface (2') of the inflating portion (2). However, if no obstacle is affected to the transmissivity of the laser beam through the protective film (4) and the other film, the position of adhering the protective film (4) is not always to be limited to the head surface only but a more widely ranging application or adherence of the protective film (4) on to the inflating portion (2) and a thicker film may be applicable.

According to the above described embodiments, even if the inflating portion (4) is broken during treatment, the broken pieces are still prevented from scattering within the blood vessel, for example, and the shrinking action is pursuantly actuated by the inflating portion itself.

Furthermore, as shown in Fig. 8, Fig. 12 and Fig. 13 with conventional balloons, it is optional to provide for reinforcing purposes a suitable number of projective rows (5) with a thicker film portion between the fixing portion (1) and the inflating portion (2), or on the end portion of inflating portion (2), or in such a manner as to separate the inflating portion into two parts.

According to the aforementioned embodiments, the thin film body (A) includes the cylindrical fixing portion (1), the inflating portion (2), the waisted portion (3), the anti-breakdown protective film (4) and the like, whereby these describe the shape of a balloon which is used with an endoscope of the kind well-known in its features and structure in the prior art.

The balloon disclosed in the aforesaid embodiments as used with an endoscope is fixed onto the end portion (b) of a conventional endoscope (B), wherein the end portion (b) has an objective lens (6), two light-guides (7), (7) (Fig. 14) which illuminate the visual field seen through the objective lens, an opening described as a channel (8) into which is inserted treatment instruments or forceps, and further openings such as an air and water supplying port (9). The end portion (b) can be moved up and down or left and right remotely within the internal organs or blood vessels it is in and, in the case of a laser beam being irradiated, the optical fiber (10) is inserted into the channel (8) and then the irradiation of laser beam is effected. It is optional to arrange a concave-convex portion (11) such as a concave row or convex row upon the outer surface of the end portion (b) for the purpose of preventing slip.

This concave-convex portion (11) is formed on the outer surface of end portion of the conventional endoscope (B) with a number of independent or continued concave rows or convex rows such as a projection or a hollow or a longitudinal row, a transverse row and a ring shape, the shape being essentially optional, but where it is still possible to fix the fixing portion (1) of the balloon coveringly upon the concave-convex portion so as to fix them together firmly.

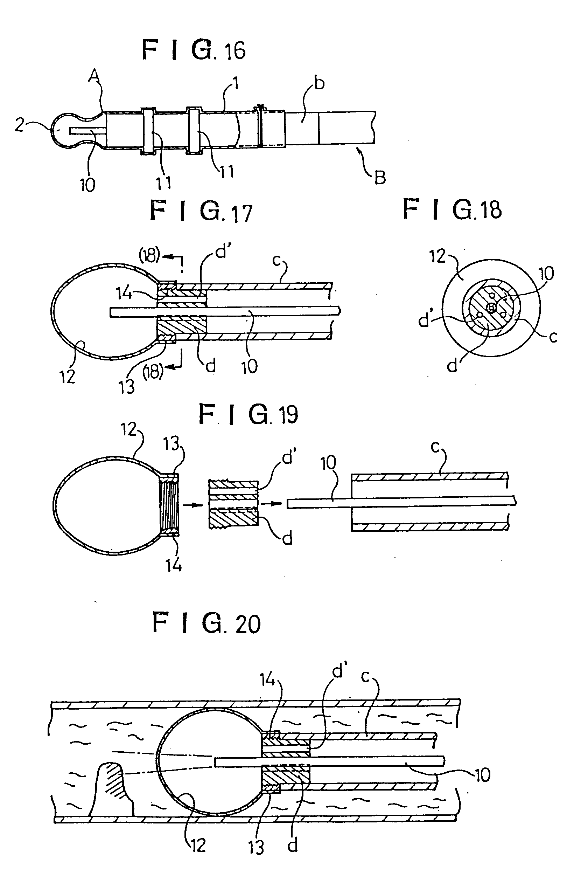

Now explaining the use of the conventional or inventive balloon with an optical fiber with reference to Fig. 17 to Fig. 20 inclusive, in this case, the inflating portion (12) is formed in the same way as with the aforementioned inflating portion (2) in which the inflating portion (12) is formed as a hollow shape by a transparent elastic thin-film which is composed of rubber latex or silicon rubber with a thickness of around 0.10--0.40 mm. The inflating portion (12) is inflated by means of injecting physiological salt solution into the hollow portion of the inflating portion (12). As a result of the inflation, the transmissivity of laser beam is improved with respect to its irradiation through the optical fiber (10).

An embodiment of the balloon as disclosed in Fig. 17 to Fig. 19 inclusively comprises a fixing ring (14) which is fixed within the interior surface of opening portion of the inflating portion (12). The ring (14) is further engaged with a support instrument of fiber tube (d) which is to be fixed detachably into the end portion of the flexible protective-pipe (c).

As shown, the fixing ring (14) is a connecting part to fix the inflating portion (12) into the end of the pipe (c), wherein an inner thread is threaded within the ring (14) for plugging into the support instrument of fiber tube (d) detachably. The outer surface of the ring (14) is plugged into the opening (13) of inflating portion (12) fixedly by a bond or the like so that it forms an integral shape with the inflating portion (12).

The features and structures of the above optical fiber (10) and its flexible protective pipe (c) are well-known in the technical field of the art. Within the flexible protective pipe (c), the optical fiber (10) is inserted through to where the fiber (10) can transmit laser beams such as YAG laser, argon laser, argon dye and the like through the balloon, whereby the end of optical fiber (10) is supported by the support instrument (d) in the end portion of the flexible protective pipe (c). The support instrument (d) is used for supporting the optical fiber (10) in fixed engagement with the interior surface of the end of the flexible protective pipe (c). The outer surface of the end of support instrument (d) is threaded for engaging with the inner thread of the fixing ring (14). Grooves or holes (d') which supply the physiological salt solution are provided in a suitable number within the support instrument (d). An adequate amount of the physiological salt solution is injected into the flexible protective pipe (c) directly, or, into the channel of the endoscope or into the air and water supply orifices indirectly, such that the solution is filled into the inflating portion (12) through said pipe (c) and holes (d').

As a result of the inflation of the portion (12), the transparency is further improved so that the transmissivity of laser beams such as YAG laser, argon laser, argon dye laser and the like will also improve. Furthermore, by inflating the portion (12), it is possible to cause a compression around bleeding portions or focuses, or to widen the isthmuses, or to block the blood flow by close contact of the portion (12) with the blood vessel walls.

Explaining now in detail the process of producing a balloon, the forming mold is dipped into a solution which combines, in relative amounts, a small quantity of powdered vulcanizing agent and powdered vulcanizing accelerator, whereby the powdered additive agents are used as sparingly as possible, liquid vulcanizing accelerator and liquid anti-oxidant, all of these agents being combined with rubber latex.

The composition of the combined solution consists of 100 WF of the rubber content of rubber latex which includes additive agents of 0.8 WF of the powdered vulcanizing agent, 0.3 WF of the powdered vulcanizing accelerator, 0.7 WF of the liquid vulcanizing accelerator and 1.0 WF of the liquid anti-oxidant, or which also includes the additive agents of 0.7--1.5 WF, but preferably 0.8--1.2 WF of the powdered vulcanizing agent, 0.2--0.5 WF, but preferably 0.3--0.4 WF of the powdered vulcanizing accelerator, 0.5--2.0 WF of the liquid vulcanizing accelerator and 0.5--2.0 WF of the liquid anti-oxidant.

The process of forming the combination comprises adding the originally separated vulcanizing agent and vulcanizing accelerator together into the rubber latex, agitating these by mixing, then adding the vulcanizing accelerator and the anti-oxidant into the rubber latex mixture of above, keeping it within a constant temperature container at 10°C to 15°C for 12 to 24 hours and, finally, keeping it in the same container at 23°C to 35°C for 24 to 48 hours to accelerate the maturing of the combined solution.

The forming mold is a so called dipping type mold for obtaining a body having a predetermined size and shape. This mold is dipped into the aforesaid combined solution for 30 to 60 seconds after having been dipped into a coagulating agent. Following this, the dipped mold is pulled up slowly so that it has a thin film of rubber latex with a thickness of approximately 0.10 to 0.40 mm formed thereon. The film adhered around the mold is then vulcanized at 80°C to 90°C for 30 minutes to produce a thin film. This film is consequently separated from the mold by using an exfoliate powder or the like. The mold is washed and dried for repeated use.

The thin-film shaped body obtained from the mold is treated with silicone emulsion for developing the transparency of the thin-film after the washing of the discharged film, and then dried at 80°C for 60 minutes. The mouth of the film is also cut off at a predetermined length. The transparent thin-film body (A) is formed such that the elastic and inflatable inflating portion (2) is arranged integrally with the end of fixing portion (1), which has a cylindrical shape with an inside diameter of approximately 1.0--17.0 mm and has an opening in the one side of the cylindrical shape.

The inflating portion (2) is transparent from the inside, and by injecting therein the physiological salt solution from the channel (8) of endoscope (B) or from the air and water supply orifices (9), the transparent inflating portion (2) is inflated to a spherical body, an inflated projecting body or a cylindrical shaped body, all of which are inflated to a predetermined size. In the above case, it may be optional as to whether or not it is formed with a larger diameter than that of the fixing portion, or a smaller or similar diameter. Furthermore, it is possible to arrange the waist portion with a smaller diameter between the inflating portion and the fixing cylindrical portion, or reinforcing rings of projected rows incorporated into the same body.

The inside diameter of the inflating portion may be selected suitably from between 0.9 mm and 35.0 mm in compliance with the diagnose or the focus to be cured. The thin-film portion of inflating portion (2) becomes thinner by means of injecting the physiological salt solution into the inflating portion and, consequently, the transparency is not only improved but the transmissibility of laser beam will also be improved.

DETAILED COMPOSITIONS

The following are examples of more detailed compositions of basic materials used to mold the inflating portion:

| PART I | |
| --- | --- |
| (1) Natural rubber latex (rubber part) (poly-1.4-isoprene) | 100 WF |
| (2) Vulcanizing agent (colloid sulphur) | 0.8 |
| (3) Vulcanizing accelerating aid (zinc oxide) | 0.3 |
| (4) Vulcanizing accelerator (Dithio-carbamate compounds) | 0.7 |
| (5) Anti-oxidant (paraffin emulsion) | 1.0 |

| PART II | |
| --- | --- |
| (1) Natural rubber latex (rubber part) (cys-1.4-poly.isoprene) | 100 WF |
| (2) Vulcanizing agent (colloid sulphur) | 1.2 |
| (3) Powdered vulcanizing accelerating aid (zinc oxide) | 0.35 |
| (4) Vulcanizing accelerator (dithio-carbamate compounds) | 0.7 |
| (5) Anti-oxidant (paraffin emulsion) | 1.0 |

PROCESS

An amount of a coagulating agent solution is prepared at normal temperatures and contains 10% of calcium nitrate methanol. The forming mold is dipped slowly into the above solution and drawn up slowly again, and then removed into the combined solution containing the natural rubber latex. After dipping into the above, the mold coated with a thin latex film is dried in the vulcanizing oven at 80°C for 30 minutes.

After being vulcanized dry, the thin latex film is peeled off from the mold by using an exfoliating powder, and the thin latex film, that is, the thin film body (A) is cured in a warm water container at 40°C to 50°C for 24 hours during which the water is circulated forcedly within the container to extract unnecessary contents within the rubber. After the curing process in the water, the thin film body (A) is additionally dipped into a solution containing 10% silicon emulsion at normal room temperature in order to improve the transparency of the thin film. The thin film body (A) is again dried for 60 minutes at 80°C in an oven to obtain a highly transparent thin film body. The relative sizes of the above body are as follows:

| Position | Film thickness (mm) | Inside diameter (mm) |
| --- | --- | --- |
| Fixing portion | 0.16 | 1.94 |
| Waisted portion | 0.22 | 1.54 |
| Globular shape portion | 0.16 | 1.81 |
| Top surface of inflating portion | 0.19 | |

The transparent thin film body (A) is not restricted to the above relative dimensions. Modifications may be made in practicing the invention and the composition modified by the use of different agents, solutions and combinations. Accordingly, it is not intended to have the invention limited to or circumscribed by the specific details of procedure, materials or proportions outlined herein above by way of example only, and the invention is susceptible to modifications according to individual preference or conditions without departing from the scope of the appended claims.

## Claims

1. A balloon for an endoscope, comprising an elastic transparent thin film body incorporating a hollow globular inflatable portion (2) formed on one end of a cylindrical fixing portion (1), characterized in that a transparent protective film body (4) is applied to the end of the inflatable portion (2) remote from the cylindrical fixing portion (1), the protective film body (4) having an elasticity similar to that of the inflatable portion (2).

2. A balloon for an endoscope according to claim 1, characterized in that the inflatable portion (2) is formed on the end of the cylindrical fixing portion (1) by means of a waist portion (3), the waist portion (3) having a film thickness greater than the film thicknesses of the inflatable portion (2) and the fixing portion. (1)

3. A balloon for an endoscope according to claim 1 or 2, characterized in that the inflatable portion (2) has a diameter smaller than that of the fixing portion (1).

## Patentansprüche

1. Ballon für ein Endoskop, mit einem elastischen, transparenten Dünnfilmkörper, der einen hohlen, kugelförmigen, aufblähbaren Abschnitt (2) aufweist, der auf einem Ende eines zylindrischen Befestigungsabschnitts (1) vorgesehen ist, dadurch gekennzeichnet, daß ein transparenter Schutzfilmkörper (4) auf dem Ende des aufblähbaren Abschnitts (2) entfernt von dem zylindrischen Befestigungsabschnitt (1) angebracht ist, wobei der Schutzfilmkörper (4) eine Elastizität aufweist, die ähnlich ist wie die des aufblähbaren Abschnitts (2).

2. Ballon für ein Endoskop nach Anspruch 1, dadurch gekennzeichnet, daß der aufblähbare Abschnitt (2) auf dem Ende des zylindrischen Befestigungsabschnitts (1) mit Hilfe eines Taillenabschnitts (3) ausgebildet ist, wobei der Taillenabschnitt (3) eine Filmdicke aufweist, die größer ist als die Filmdicke des aufblähbaren Abschnitts (2) und des Befestigungsabschnitts (1).

3. Ballon für ein Endoskop nach Anspruch 1 oder 2, dadurch gekennzeichnet, daß der aufblähbare Abschnitt (2) einen kleineren Durchmesser aufweist als der Befestigungsabschnitt (1).

## Revendications

1. Ballon pour endoscope, comprenant un corps en fine pellicule transparente élastique qui incorpore une partie gonflable globulaire creuse (2) formée sur une extrémité d'une partie de fixation cylindrique (1), caractérisé en ce qu'un corps (4) en pellicule protectrice transparente est appliqué à l'extrémité de la partie gonflable (2) distante de la partie de fixation cylindrique (1), le corps (4) en pellicule protectrice présentant une élasticité similaire à celle de la partie gonflable (2).

2.  Ballon pour endoscope selon la revendication 1, caractérisé en ce que la partie gonflable (2) est formée sur l'extrémité de la partie de fixation cylindrique (1) au moyen d'une partie étranglée (3), la partie étranglée (3) ayant une épaisseur de la pellicule supérieure aux épaisseurs des pellicules de la partie gonflable (2) et de la partie de fixation (1).

3.  Ballon pour endoscope selon la revendication 1 ou 2, caractérisé en ce que la partie gonflable (2) a un diamètre inférieur à celui de la partie de fixation (1).

F I G.1

F I G.2

F I G.3

F I G.4

F I G.5

# F I G.6

# F I G.7

# F I G. 8

# F I G. 9

F I G.11

F I G.10

F I G. 12

F I G. 13

F I G.14

F I G.15

EP 0 288 576 B1

F I G. 16

F I G. 17

F I G. 18

F I G. 19

F I G. 20

14